# EUROPEAN PATENT APPLICATION

(11) **EP 3 284 827 A1**
(43) Date of publication of application: **21.02.2018**
(21) Application number: 16184189.5
(22) Date of filing: 15.08.2016
(51) Int. Cl.: C12P 3/00, B01D 53/75, B01D 53/78, B01D 53/84, C12M 1/00, C12M 1/40, C12N 1/12, C12P 7/64

(54) **PRODUCTION OF ALGAE USING CO2-CONTAINING GAS**

(71) Applicant: Nederlandse Organisatie voor toegepast- natuurwetenschappelijk onderzoek TNO, 2595 DA 's-Gravenhage (NL)
(72) Inventor: Goetheer, Earl Lawrence Vicent, 2595 DA 's-Gravenhage (NL); Könst, Paul Mathijs, 2595 DA 's-Gravenhage (NL); Hernandez Mireles, Ileana, 2595 DA 's-Gravenhage (NL); van Os, Peter, 2595 DA 's-Gravenhage (NL); van der Stel, Robrecht Wouter, 2595 DA 's-Gravenhage (NL)
(74) Representative: V.O.

(57) **Abstract**

The invention is directed to a method for using a gaseous feed stream comprising CO₂ as a feed stream for growing algae, said method comprising a) contacting said gaseous feed stream with an absorption liquid comprising carbonic anhydrase in an absorption module to produce a purified gas stream and a liquid feed stream comprising bicarbonate; b) contacting part of the liquid feed stream and/or part of the gaseous feed stream comprising CO₂ with microorganisms to generate carbonic anhydrase and c) contacting part of the liquid feed stream with an algae culture to produce algae-containing biomass. Another aspect of the invention is directed to an apparatus for sequestration of CO₂ by using algae, said apparatus comprising two coupled circulation systems that each comprise at least one bioreactor.

## Description

The invention is in the field of CO₂ capture and sequestration, as well as in the field of producing algae. The invention is in particular directed to using CO₂-containing gas to grow algae for biomass production as well as to an apparatus suitable for carrying out this production process.

To reduce the amount of CO₂ emission to the atmosphere, CO₂ contained in gaseous streams, such as flue gases, can be captured and sequestrated from these streams. A number of different methods are known to achieve this. Particular methods of interests are those that use algae for CO₂ sequestration. These methods have the concomitant advantage that the algae proliferate on CO₂ and can serve as a biomass source for the production of valuable products. Conventional methods include directly contacting a algae culture with gaseous CO₂. This however has a limited efficiency, in particular when the process is carried out at large scale. This issue has been addressed by a combined enzymatic CO₂ capture and subsequent sequestration by algae.

WO 2013/022348 describes the capture of CO₂ by carbonic anhydrase by conversion of the CO₂ into bicarbonate. The bicarbonate is subsequently fed to algae. To limit the waste of enzymes, a filtering system and recycling of the enzymes are provided. However, this has only a limited efficiency and although carbonic anhydrase is an enzyme and acting as a catalyst theoretically not consumed, the carbonic anhydrase in practice loses reactivity (efficiency) due to decomposition. It is therefore required to replenish the carbonic anhydrase in the absorption unit. Conventionally, this is achieved by adding carbonic anhydrase that is processed in a separate process but this is overall less efficient.

A first aspect of the present invention is accordingly directed to a method for using a gaseous feed stream comprising CO₂ as a feed stream for growing algae, said method comprising:
a) contacting said gaseous feed stream with an absorption liquid comprising carbonic anhydrase in an absorption module to produce a purified gas stream and a liquid feed stream comprising bicarbonate;
b) contacting part of the liquid feed stream and/or part of the gas stream comprising CO₂ with microorganisms to generate carbonic anhydrase;
c) contacting part of the liquid feed stream with an algae culture to produce algae-containing biomass.

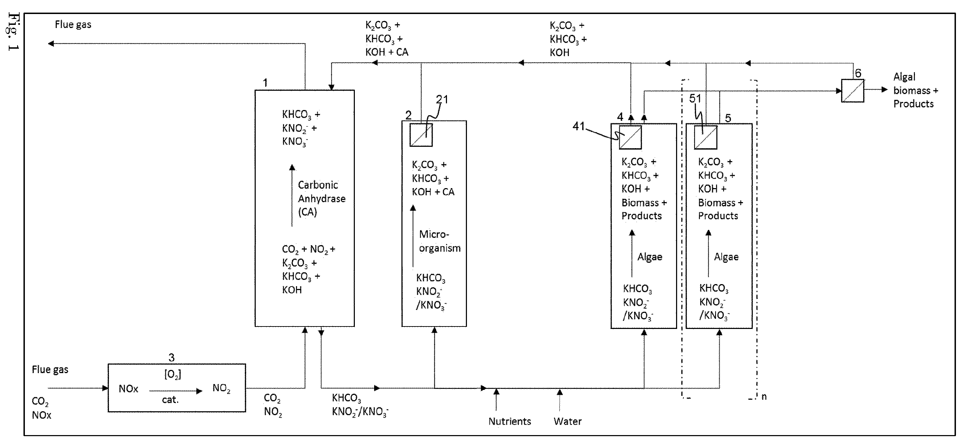

Figure 1 is a schematic representation of a particular embodiment of the present invention.

A particular advantage of the method of the present invention is that the integrated production of the carbonic anhydrase reduces the necessity for further carbonic anhydrase addition which may be produced off-site. This greatly simplifies the overall process and increases its efficiency. An additional advantage of the present method is that the gaseous feed stream is either directly or indirectly, used to produce the carbonic anhydrase. This reduces the amount of CO₂ emission as well.

The gaseous feed according to the present invention typically comprises flue gas, *e.g.* flue gas originating from an electrical power plant. Flue gas generally comprises, besides CO₂, gaseous NOₓ. NOₓ includes nitrogen oxides (e.g. nitrogen monoxide NO) that are considered toxic and harmful for the environment and are therefore preferably removed from the gaseous feed stream as well. It has surprisingly been found that by oxidization the NOₓ to NO₂, this NOₓ can however be advantageously used in the present invention. Therefore, in a preferred embodiment of the present invention, the gaseous feed stream further comprises NOₓ and said method further comprises oxidation of said NOₓ with a catalyst to form an oxidized gaseous feed stream comprising NO₂ and the CO₂. NOₓ can be oxidized by known catalysts. Examples thereof include palladium, platina, titanate (TiO₂), cobalt, zinc, nickel, manganese, and combinations thereof. Suitable catalysts are well known in the art, see for example the catalyst materials described in US8557203, which is incorporated herein. Instead of or in addition to using the oxidation catalyst, the oxidation can be carried out with oxidation agents such as ozone (see *e.g*. US5985223)

In this particular embodiment, the oxidized gaseous feed stream is subsequently used in step a) of the present invention.

In case the gaseous feed stream comprises CO₂ and NOₓ and is oxidized to the oxidized gaseous feed stream, an oxidation catalyst that is capable of forming nitrite (NO₂⁻) and/or nitrate (NO₃⁻) from NO₂ is preferably present in the absorption liquid. Examples of oxidation catalysts known for the conversion of NO₂ into NO₃⁻ and NO₂⁻ are for instance Fe²⁺. Nagase et al. (Characteristics of Biological NOx Removal from Flue Gas in a Dunaliella Tertiolecta Culture System. J. Ferment. Bioeng. 83 (1997)461-465) reported 60% NOx (25 to 500 ppm) removal in presence of the algae D. *tertiolecta* and 30% when only led through cell fee medium. It was assumed that photochemical oxidation took place, catalyzed by Fe²⁺.

Accordingly, the liquid feed stream may further comprise nitrite and/or nitrate which can be used to feed and culture the algae. Thus the conventionally undesired NOₓ may advantageously be used for the present invention to in fact improve growth of the algae.

Carbonic anhydrase (also referred to as carbonate anhydrase; EC 4.2.1.1) is known to catalyze the hydration of CO₂ (see for instance WO2013/022348 which is incorporated herein in its entirety). The generation of the carbonic anhydrase in the present invention is achieved by contacting part of the liquid feed stream and/or part of the gas stream comprising CO₂ with microorganisms. Examples of microorganisms that can be used to generate carbonic anhydrase are for instance bacteria as described by A. Di Fiori et al. (Int. J. Mol. Sci. 16 (2015) 15456-15480) and the references cited therein.

Carbonic anhydrase is widespread among the entire prokaryotic and eukaryotic domain (see Shekh et al.; Recent Advancements in Carbonic Anhydrase-Driven Processes for CO2 Sequestration: Minireview; Crit. Rev. Environ. Sci. Technol. 2012, 42 (14) 1419-1440). Carbonic anhydrase is either localized inside the cell, or can be secreted outside. External carbonic anhydrase is located outside or associated with the periplasmic cell membrane. External carbonic anhydrase is expressed under certain conditions, especially when CO₂ supplements are limited to the cell (see Lam et al.; Current Status and Challenges on Microalgae-Based Carbon Capture. Int. J. Greenh. Gas Control 10 (2012) 456-469). A stream of the carbonic anhydrase generated in step b) of the invention is typically directly fed to the absorption module. Alternatively, the stream of carbonic anhydrase can first be combined with a basic liquid stream that is additionally produced in step c), after which the combined stream comprising carbonic anhydrase and the basic liquid are fed to the absorption module.

The basic liquid stream is the result of contacting the algae and the liquid feed stream comprising the bicarbonate. The algae process the bicarbonate present in the liquid feed stream resulting in an increase of the pH of the culture, typically due to the production of hydroxide. The pH of the algae culture is preferably maintained at constant levels. Preferred pH values range from 7 to 10. This can be achieved by a selective (essentially cell-free) outflow of a basic liquid stream from the algae culture. A filter may be used to prevent the outflow of algae together with the basic liquid. Due to the outflow of the basic liquid and subsequent replenishment with the relatively acidic feed stream, the pH value in the algae culture may be kept constant.

The basic liquid stream is preferably recycled to the absorption module, since due to the absorption of CO₂, the pH of the absorption liquid decreases in the absorption module. Preferably the absorption liquid has a pH value ranging from 6-8. Hence, by providing an outflow and recycling of the basic liquid stream, the pH values of both the algae culture and the absorption liquid can be controlled. This is particularly beneficial for the overall efficiency of the process since it prevents the requirement of further addition of base and/or acid.

Typically, steps b) and c) are carried out in separate bioreactors. Step b) may for instance be carried out in a first bioreactor and step c) is carried out in a second bioreactor. Each bioreactor generally comprises a separation module (e.g. a filter or a membrane) to separate the microorganisms and/or algae from the outflowing stream, e.g. the stream of carbonic anhydrase and/or the liquid basic stream. The bioreactor used for each step can comprises a plurality of parallel bioreactors. This greatly facilitates flexible scaling of the process, depending on the amount of the gaseous feed stream to be processed.

The culture of algae can further be fed with water and nutrients besides with the bicarbonate and optional nitrates and/or nitrites. For instance the following nutrients may be present: sodium nitrate, calcium chloride, magnesium sulfate, potassium phosphate (mono- and dibasic), sodium chloride, sodium EDTA, iron (III) chloride, manganese (II) chloride, zinc (II) chloride, cobalt (II) chloride, sodium molybdate, vitamin B12, biotin, thiamine, and combinations thereof. These additional water and nutrients can be supplied to the liquid feed stream before this stream is contacted with the algae.

The algae culture preferably comprises *Spirulina platensi* and/or *Neochloris oleoabundans.* Other examples of strains are Chlorella, Chlamydomonas, Nannochloropsis, Botryococcus, *etc.* The culture is capable of converting the bicarbonate into valuable compounds such as ß-carotene, anti-oxidants, lipids and fatty acids can be produced by the algae. The produced algae-containing biomass thus comprises a number of valuable compounds. In a preferred embodiment of the present invention, the method further comprises a step of harvesting the algae and subsequent extraction of the valuable compounds. These valuable compounds can be used in applications such as biofuel and cosmetics.

A further aspect of the present invention is an apparatus that is especially adapted for the method according to the present invention.

The apparatus according to the present invention comprises a first fluid circulation system comprising an absorption module (1) and a first bioreactor (2) downstream of the absorption module. The first bioreactor is adapted to culture microorganisms producing carbonic anhydrase and comprises a filter (21) positioned downstream in the reactor such that the microorganisms are essentially retained in the first bioreactor and an outflow stream comprises carbonic anhydrase can be obtained that is essentially cell-free.

The apparatus further comprises a second fluid circulation system, that is connected to the first circulation system and extents directly from the absorption module (1) or downstream of the absorption module and upstream of the first bioreactor (2), to the absorption module or to the first circulation system downstream of the first bioreactor and upstream of the absorption module. The second fluid circulation system comprises a second bioreactor (4) that is adapted to culture algae. The second bioreactor (4) comprises a filter (41) positioned downstream such that the algae are essentially retained in the second bioreactor and an basic outflow stream that is essentially cell-free can be obtained and the taken volume be replaced with relatively acidic feed stream with the aim to maintain a desired pH value in the bioreactor.

In the case that a plurality of parallel bioreactors are used for the algae culture, the apparatus further comprises additional fluid circulation systems connected parallel to the second fluid circulation system. Each additional fluid circulation system comprises an additional bioreactor (5) similar to as the second bioreactor (4) which additional bioreactor (5) accordingly comprises an additional filter (51). The additional fluid circulation system preferably comprises a valve upstream of the additional bioreactor such that the additional fluid circulation system can flexibly be used depending on the demand of capacity.

In a preferred embodiment of the apparatus, the apparatus further comprises a catalyst module (3) adapted for oxidizing NOₓ. The catalyst module is generally connected to the absorption module via a gas transport system such as a tube.

## Claims

1. Method for using a gaseous feed stream comprising CO₂ as a feed stream for growing algae, said method comprising:
a) contacting said gaseous feed stream with an absorption liquid comprising carbonic anhydrase in an absorption module to produce a purified gas stream and a liquid feed stream comprising bicarbonate;
b) contacting part of the liquid feed stream and/or part of the gaseous feed stream comprising CO₂ with microorganisms to generate carbonic anhydrase;
c) contacting part of the liquid feed stream with an algae culture to produce algae-containing biomass.

2. Method according to claim 1, wherein said gaseous feed stream further comprises NOₓ and said method further comprises oxidation of NOₓ, preferably oxidation with a catalyst, to form an oxidized gaseous feed stream comprising NO₂ and CO₂ that is contacted with the absorption liquid in step a); and wherein the liquid feed stream further comprises NO₂⁻ and NO₃⁻.

3. Method according to any of the previous claims, wherein step b) further comprises producing a stream of carbonic anhydrase that is fed to the absorption module.

4. Method according to any of the previous claims, wherein steps b) and c) are carried out in separate bioreactors, step b) is carried out in a first bioreactor and step c) is carried out in a second bioreactor.

5. Method according to any of the previous claims, wherein the pH of the absorption liquid is controlled by the addition of a basic liquid stream that is additionally produced in step c).

6. Method according to any of the previous claims, wherein the pH of the algae culture is controlled by the outflow of the basic liquid stream.

7. Method according to any of the previous claims, wherein the microorganisms comprise cyanobacteria, algae, or a combination thereof.

8. Method according to any of the previous claims, wherein step c) is carried out in a plurality of parallel bioreactors comprising algae.

9. Method according to any of the previous claims, wherein additional water and nutrients are added to the liquid feed stream before it is contacted with the algae.

10. Method according to any of the previous claims, wherein the gaseous feed comprises flue gas, preferably flue gas originating from an electrical power plant.

11. Apparatus for sequestration of CO₂ by using algae, preferably by using a method according to any of the previous claims, said apparatus comprising:
- a first fluid circulation system comprising
- an absorption module comprising a gas inlet and;
- downstream of the absorption module a first bioreactor comprising a filter adapted to retain microorganisms in the first bioreactor;
- a second fluid circulation system, that is connected to the first circulation system and extents from the absorption modules or downstream of the absorption module and upstream of the first bioreactor to upstream of the absorption module or to the first circulation system downstream of the first bioreactor and upstream of the absorption module, said second fluid circulation system comprising;
- a second bioreactor that is adapted to culture algae, said second bioreactor comprises an outlet comprising a filter adapted to enable a liquid outflow while retaining the algae in the second bioreactor.

12. Apparatus according to claim 11, further comprising an oxidation module comprises a catalyst module adapted for oxidizing NOₓ connected to the gas inlet of the absorption module via a gas transport system such as a tube.
